# EUROPEAN PATENT APPLICATION

(11) **EP 1 034 792 A1**
(43) Date of publication of application: **13.09.2000**
(21) Application number: 99104803.4
(22) Date of filing: 11.03.1999
(51) Int. Cl.: A61K 39/09, A61K 39/385, A61K 39/39

(54) **Intranasal delivery of pneumococcal polysaccharide vaccines**

(71) Applicant: PASTEUR MERIEUX SERUMS ET VACCINS, 69007 Lyon (FR); Jonsdottir, Ingileif, 105 Reykjavik (IS)
(72) Inventor: Jonsdottir, Ingileif, 105 Reykjavik (IS)
(74) Representative: Ayroles, Marie-Pauline

(57) **Abstract**

The invention relates to a method for preventing against diseases induced by *Streptococcus pneumoniae* infections, which comprises mucosally administering to a patient in need a *S. pneumoniae* capsular polysaccharide. This latter may be conjugated or not and is preferably mixed with a mucosal adjuvant such as cholera toxin, *E. coli* heat-labile toxin or Rhinovax™. A preferred route of administration is the intranasal route.

## Description

The invention relates to a method for preventing mammals against *Streptococcus pneumoniae* infections which comprises mucosally administering to a patient in need, a S. *pneumoniae* capsular polysaccharide.

The mucosal surfaces of respiratory, genitourinary and gastrointestinal tracts are covered by a specialized epithelium, which creates an efficient physical barrier against environmental pathogens. However, a majority of bacterial and viral infections directly affect or enter the body through mucosal surfaces and colonization at these sites is often the first step in pathogenesis. *S. pneumoniae* is a major pathogen, which enters the body through the respiratory mucosa and may cause serious infections such as pneumonia, bacteremia and meningitis, especially in elderly people with a variety of chronic diseases, and in young children. It is also a common cause of mucosal infections like otitis media and sinusitis.

The pneumococcus is surrounded by capsular polysaccharides, which are the main virulence factors and protect the pneumococci from defense mechanisms of the host which largely depends on opsonophagocytosis mediated by antibodies and complement (phagocytosis of bacteria opsonized by type-specific IgG antibodies and complement). Capsular polysaccharides can induce antibody production in the absence of T-cell help and are therefore classified as thymus-independent antigens type 2 (TI-2). It is thought that the TI-2 antigens only activate mature B cells, which may be one reason why infants respond poorly to polysaccharide antigens. Conjugation of polysaccharides to proteins makes them immunogenic in infants. The immunogenicity of such pneumococcal polysaccharide conjugate vaccines is assumed to be related to their thymus-dependent-like character, although the mechanism is not known in detail.

Systemic vaccination has lead to a significant reduction in morbidity and mortality caused by a variety of pathogens, were protection has been shown to correlate with serum IgG antibody titers. An injectable pneumococcal vaccine containing polysaccharides of 23 serotypes and already on the market, is efficient to protect from adult invasive infections and exhibits some transitory potency in children over seven months. Nevertheless, systemic immunization does not induce mucosal immune responses, which may be important against infections of the respiratory tract.

Wu et al, J. Infect. Dis. (1997) 175 : 839 has shown that pneumococcal polysaccharide of serotype 6B conjugated to tetanus toxoid is able to elicit protection against nasopharyngeal carriage when administered intranasally, in the presence of a mucosal adjuvant. However, there is no definitive correlation between carriage and infection. Indeed, some serotypes such as serotype 23F, are seldomly found in the nasopharynx and still have a high incidence on the diseases induced by *S. pneumoniae.* Further, Wu et al does not study the type of immune response elicited by intranal administration of the conjugate and therefore does not suggest that the immune response can be sufficient to protect against infection, although it protects against carriage.

Flanagan & Michael, Vaccine (1999) 17 : 72 reports that oral immunization with a S. *pneumoniae* polysaccharide conjugate induces serum IgG antibodies against type specific polysaccharides. However, no local secretory IgA (S-IgA) anti-polysaccharide response was detected. S-IgA is the major antibody isotype at mucosal surfaces and may inhibit adherence and invasion of mucosal pathogens and neutralize virulence enzymes and toxins. Accordingly, the absence of S-IgA teaches against the possibility of achieving protection against *S. pneumoniae* using the mucosal route.

The potential of mucosal immunization to protect against pneumococcal infections, has nevertheless been investigated and it has now been found that protection may be achieved.

Therefore, the invention relates to the use of a *S. pneumoniae* capsular polysaccharide in the preparation of a medicament to be mucosally administered for preventing against diseases induced by *S. pneumoniae* infections. Alternatively, the invention also relates to a method for preventing mammals against diseases induced by *S. pneumoniae* infections, which comprises mucosally administering a *S. pneumoniae* capsular polysaccharide to a mammal in need.

A preferred route of administration is the intranasal route. It is indicated that the medicament is intranasally administered so that it be essentially delivered to the respiratory tract *i.e.* the nasal membrane or the pulmonary tract. As a result of this, it is possible to stimulate the mucosal associated lymphoid tissue (MALT), in particular the bronchial associated lymphoid tissue (BALT), that drain the mucosal, *e.g.* nasal or bronchial, membranes that host *S. pneumoniae.* Conventional devices useful for targeting a pharmaceutical product to the respiratory tract, are commonly available and it is within the skills of a man in the art to select the appropriate device for a given formulation according *e.g.* the volume, the ingredients or the age of the recipient.

Polysaccharides may be extracted from *S. pneumoniae* and purified according to conventional processes. They may be used as such or depolymerized in order to produce polysaccharides having a mean molecular weight lower than that of the polysaccharides originally extracted. Fragmentation may be conventionally achieved, in particular as described in WO 93/7178, incorporated herewith by reference.

For use in the present invention, the polysaccharide may be conjugated or not to a carrier polypeptide. By "conjugate" is meant a compound in which the polysaccharide is covalently linked to a carrier polypeptide. By "polypeptide" is meant any chain of amino acids, regardless of the size or post-translational modification. Typically, a polypeptide may be composed of *e.g.* six amino acids or more. A carrier is defined as being a polypeptide that is able to convert, upon conjugation, a T-independent antigen to a T-dependent antigen, allowing for isotype switching and the generation of long term memory. Examples of carrier polypeptides include the *tetanus* toxoid, the *diphtheriae* toxoid, a non-toxic CRM197 mutant of *diphtheriae* toxin, the *Neisseria meningitidis* class 1 or 2/3 outer membrane protein, any variant, analog or fragment thereof that retains the carrier property. Polysaccharides may be conjugated to carrier polypeptides according to conventional methods.

For use in the present invention, the polysaccharide may be combined or not with a mucosal adjuvant. Suitable mucosal adjuvants include the cholera toxin (CT), the *E. coli* heat labile toxin (LT), the *pertussis* toxin (PT) or a subunit thereof such as CTB or LTB. These toxins may be native or recombinantly produced. CT or CTB may be prepared or purified as described in Mekalanos et al, Infect. Immun. (1978) 20 : 552, Holmgren et al, Nature (1977) 269 : 602, Tayot et al, Eur. J. Biochem. (1981) 113 : 249 or US patent N° 5,666,837. LT or LTB may be prepared or purified as described in Clements & Finkelstein, Infect. Immun. (1979) 24 : 760, Clements et al, Vaccine (1988) 6 : 269 or in European patent aplication 416 505. Mutant toxins with reduced toxicity may also be used as adjuvant. They include CT Ser-61-Phe as described in Yamamoto et al, J. Immunol. (1998): 4116 and Yamamoto et al, PNAS (1997) 94 : 5267 ; LT Arg-7-Lys as described in WO 95/17211 ; LT Arg-192-Gly as described in WO 96/06627 ; LT Ser-63-Lys, Arg-192-Gly as described in WO 97/02348 ; LT Ala-72-Arg as described in WO 98/18928 ; and PT Arg-9-Lys, Glu-129-Gly as described in WO 95/09649 and WO 95/34323. All the point mutations are localized in subunit A (CT or LT) or S (PT) of the toxins. Other suitable adjuvant formulations are described in WO 94/17827, especially in claim 1 of WO 94/17827 as published. RhinoVax™ is one of these formulations. It is composed of caprylic/capric glycerides dissolved in polysorbate 20 and water.

Pneumococcal polysaccharides may be of any serotype. As a matter of example, serotypes 1, 3, 4, 5, 6B, 7F, 9V, 14, 18C, 19F and 23F are cited. One or several polysaccharides may be concomitantly administered by the mucosal route. In particular, the medicament *i.e.,* the vaccine for mucosal administration may contain several polysaccharides, each of particular serotype.

The polysaccharides, conjugated or not, may conventionally be used in the preparation of the medicament *e.g.,* vaccine. In particular, the polysaccharides may be formulated with a diluent or a pharmaceutically acceptable carrier *e.g.,* a buffer or a saline. The vaccine may additionally contain usual ingredients such as a stabiliser or as already mentioned above, a mucosal adjuvant. In a general manner, these products are selected according to standard pharmaceutical practices as described in *Remington's Pharmaceutical Sciences,* a reference book in the field.

In a vaccination protocol, the vaccine may be administered by the mucosal route, as a unique dose or preferably, several times *e.g.,* twice, three or four times at week or month intervals, according to a prime/boost mode. The appropriate dosage depends upon various parameters, including the number of valencies contained in the vaccine, the serotypes of the polysaccharides and the age of the recipient. It is indicated that a vaccine dose suitably contain per valency, from 0.5 to 100 µg, preferably from 1 to 50 µg, more preferably from 1 to 10 µg of polysaccharide. A dose is advantageously under a volume of from 0.1 to 2 ml.

The vaccination protocol may be a strict mucosal protocol or a mix protocol in which the priming dose of the vaccine is administered by the mucosal *e.g.,* intranasal route and the boosting dose (s) is (are) parenterally administered or *vice versa.*

The invention is further illustrated in the examples hereinafter, by reference to the following figures.

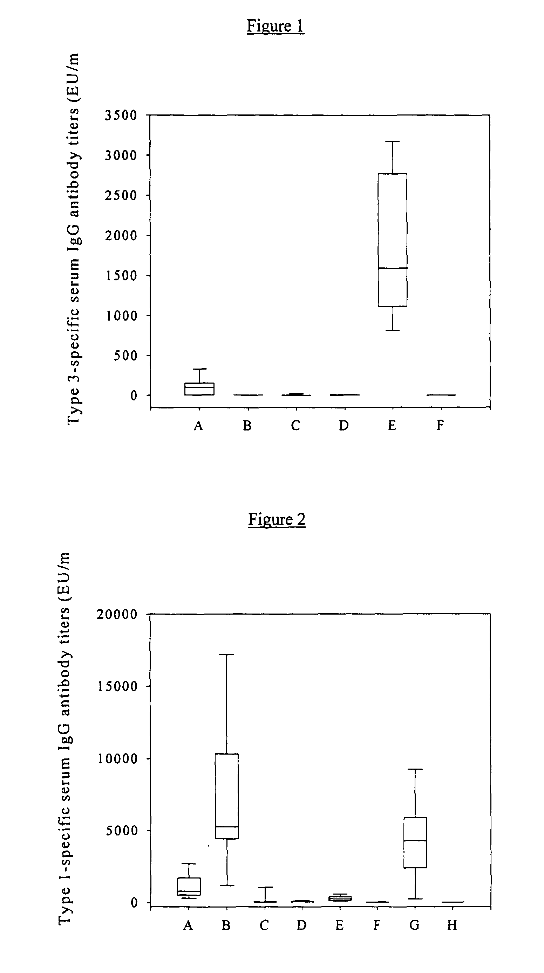

Figure 1 shows type 1-specific serum IgG antibody titers (EU/ml) after immunization with PNC-1 and PPS-1. The box-plot shows the median value with 25th-75th percentiles and the error bars indicate 5th-95th percentiles. Groups of mice are reported as follows. A: PNC-1 (0.5 µg) in RV i.n., B: PNC-1 (2.0 µg) in RV i.n., C: PPS-1 (2.0 µg) in RV i.n., D: PNC-1 (0.5 µg) in saline i.n., E: PNC-1 (2.0 µg) in saline i.n., F: PPS-1 (2.0 µg) in saline i.n., G: PNC-1 (0.5 µg) in saline s.c., H: Non-immunized control.

Figure 2 shows type 3-specific serum IgG antibody titers (EU/ml) after immunization with PNC-3 and PPS-3. Box-plot is as described for figure 1. Groups of mice are reported as follows. A: PNC-3 in RV i.n., B: PPS-3 in RV i.n., C: PNC-3 in saline i.n., D: PPS-3 in saline i.n., E: PNC-3 in FCA/FIA i.p., F: Non-immunized control.

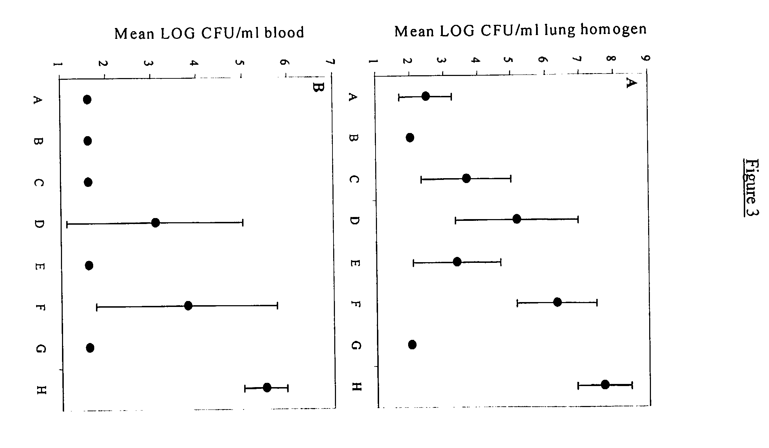

Figure 3 shows the pneumococcal density (LOG mean ± SD) in lungs (Fig. 3A) and blood (Fig. 3B) in groups of mice 24 hours after i.n. challenge with serotype 1. Each group is represented by one dot (n=10). Groups of mice are reported as follows. A: PNC-1 (0.5 µg) in RV i.n., B: PNC-1 (2.0 µg) in RV i.n., C: PPS-1 (2.0 µg) in RV i.n., D: PNC-1 (0.5 µg) in saline i.n., E: PNC-1 (2.0 µg) in saline i.n., F: PPS-1 (2.0 µg) in saline i.n., G: PNC-1 (0.5 µg) in saline s.c., H: Non-immunized control.

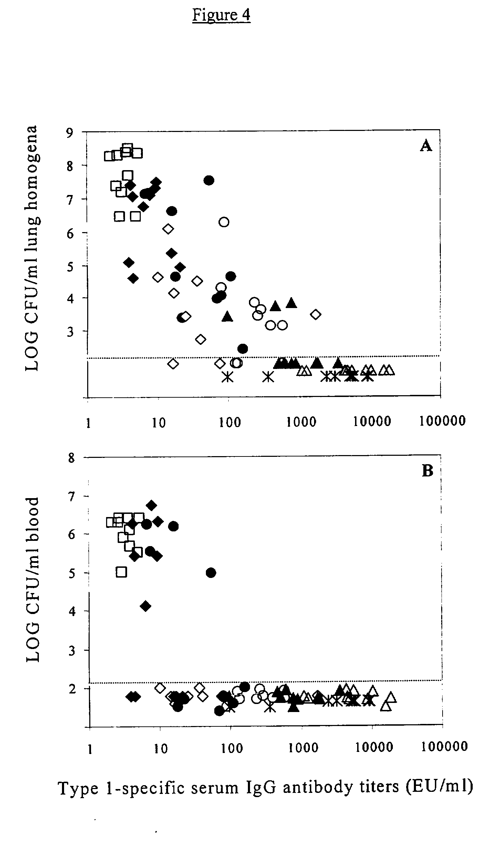

Figure 4 shows the relationship between pneumococcal lung infection (Fig. 4A) and bacteremia (as LOG CFU) (Fig. 4B) and type 1-specific IgG antibody titers in serum (EU/ml). Each symbol represent one mouse. Groups of mice are reported as follows. PNC-1 (0.5 µg) in RV i.n. (▲), PNC-1 (2.0 µg) in RV i.n. (Δ), PPS-1 in RV i.n. (◇), PNC-1 (0.5 µg) in saline i.n. (●), PNC-1 (2.0 µg) in saline i.n. (○), PPS-1 in saline i.n. (◆), PNC-1 (0.5 µg) in saline s.c. (∗), Non-immunized control (□). Dotted lines represent the detection limits for CFU.

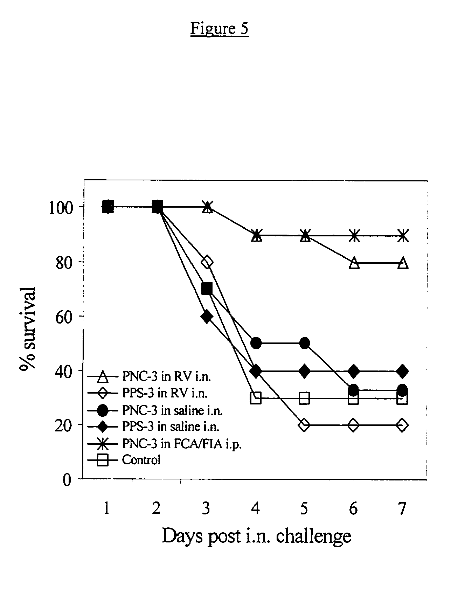

Figure 5 shows the survival of mice (n=10 per group) after intranasal challenge with pneumococci of serotype 3.

### Examples : Protection against Streptococcus pneumoniae serotypes 1 and 3

### Materials and methods

### Mice :

Six week old female outbred NMRI mice obtained from Gl. Bomholtgård Ltd, Ry, Denmark were housed under standard conditions with regulated daylength and kept in cages with free access to commercial pelleted food and water.

### Vaccines and adjuvants :

Experimental tetanus-toxoid conjugated polysaccharide vaccines (PNC's) were produced by Pasteur Merieux Connaught, Marcy l'Etoile, France. Pneumococcal polysaccharides (PPS) were purchased from American Type Culture Collection (ATCC, Rockville, MD). For intranasal (i.n.) immunization, the PNC or PPS were diluted in saline, or mixed with 20% RhinoVax (RV), a mucosal adjuvant based on caprylic/capric glycerides dissolved in polysorbate 20 and water produced at the Department of Pharmacy, University of Iceland, Reykjavik, Iceland. For parenteral immunization, PNC-1 was diluted in saline, but PNC-3 was emulsified with 50% Freund's adjuvants (FA; Sigma Chemical Co., St. Louis, Mo), complete (FCA) for primary and incomplete (FIA) for booster.

### Immunization :

The mice, 10 per group, were immunized with 0.5 µg or 2.0 µg of PNC or PPS. For i.n. immunization, 10 µl vaccine solution in RV or saline was slowly delivered into the nares of mice sedated by subcutaneous (s.c.) injection with Hypnorm (Jansen Pharmaceutica, Beerse, Belgium). For parenteral immunization, PNC-1 in saline was injected s.c. in the scapular girdle region and PNC-3 in FA was injected intraperitoneally (i.p.). All groups received a booster with the same dose and route 4 weeks after the primary immunization. Non immunized mice were used as controls.

### Blood- and saliva sampling for antibody measurements :

The mice were bled from retro-orbital sinus 15 days after boosting, serum isolated and stored at -70°C. Saliva was collected from each mouse by insertion of absorbent polyfiltronics sticks (5x2mm) to the mouth. After five minutes, the sticks were transferred to phosphate-buffered saline, pH 7.4 (PBS) containing 10.0 µg/ml protease inhibitor (Aprotinin, Sigma) to prevent proteolysis of antibodies. The dissolved saliva was pooled for each group of mice and stored at -70°C.

### Antibodies to PPS :

Specific IgM, IgG and IgAantibodies to PPS were determined by enzyme-linked immunosorbent assay (ELISA) designed according to the standardized ELISA protocol (Workshop at Centers for Disease Control, CDC, Atlanta, 1996) with few modifications. Microtiter plates (MaxiSorp, Nunc AS, Roskilde, Denmark) were coated with 10 µg polysaccharide of serotypes 1 and 3 (ATCC) per ml PBS and incubated for 5 hours at 37° C. For neutralization of antibodies to cell wall polysaccharide (CWPS; Statens Serum Institute, Copenhagen, Denmark), serum samples and standards were diluted 1:50 in PBS with 0.05% Tween-20 (Sigma) and incubated in 500 µg/ml CWPS for 30 minutes at room temperature. The neutralized sera were serially diluted and incubated in PPS-coated microtiter plates at room temperature for 2 hours.
For detection, horseradish peroxidase (HRPO) conjugated goat antibodies to mouse IgG (Caltac Laboratories, Burlingame, CA.), IgM or IgA (Sera-Lab, Sussex, England) were used. The conjugates were diluted 1:5000 in PBS-Tween and incubated for 2 hours at room temperature. For development, 3,3',5,5'- tetramethylbenzidine (TMB) peroxidase (Kirkegaard & Perry Laboratories, Gaithersburg, MD) was incubated for 10 minutes according to the manufacturers instructions and the reaction stopped by 0.18 M H₂SO₄. The absorbance was measured at 450 nm in an ELISA spectrophotometer (Titertek Multiscan® Plus MK II; Flow Laboratories, Irvine, UK). Reference serum obtained from Pasteur Mérieux Connaught was included on each microtiter plate for calculation of the titers expressed in Elisa Units (EU) per ml. The titers of the reference sera (EU/ml) corresponded to the inverse of the serum dilution giving an O.D. = 1.0.
The assays were performed at room temperature. All sera were tested in duplicates and 100-µl volumes were used in all incubation steps with three washings with PBS-Tween after each step.

### Pneumococci:

The bacteria were cultured as follows. *S. pneumoniae* of serotypes 1 and 3 (ATCC), maintained in tryptoset broth + 20% glycerol at -70°C, were plated on blood agar (DIFCO laboratories, Detroit, MI) and incubated at 37°C in 5% CO₂ over night. Isolated colonies were transferred to a heart infusion broth (DIFCO laboratories) with 10% horse serum, cultured at 37°C to log-phase for 3.5 hours and resuspended in 0.9% sterile saline. Serial 10-fold dilutions were plated on blood agar to determine inoculum density.

### Pneumococcal challenge :

The challenge experiments were performed two days after the mice were bled. The animals were anaesthetized with pentobarbitone sodium BP (50 mg/kg, Icelandic Pharmaceuticals, Reykjavik, Iceland) injected i.p. and challenged i.n. with 50 µl of bacterial suspension. To evaluate bacteremia, blood was collected from the tail vein at various time points after challenge and plated on blood agar for culturing at 37°C in 5% CO₂ over night. Bacteremia was determined as colony forming units (CFU) per ml blood. When the mice were sacrificed, the lungs were removed and homogenized in sterile 0.9% saline and serial dilutions plated on blood agar including Staph/Strep selective supplement containing nalidixic acid and colistin sulphate (Unipath Ltd., Hamshire, England). Pneumococcal lung infection was determined as CFU/ml lung homogenate. Depending on the first dilution used, the detection limit was 2.2 CFU/ml lung homogenate and 1.6 CFU/ml blood.

### Statistical analysis :

A nonparametric t-test (Mann-Whitney on ranks) and Chi-square test were used for comparison between groups. Correlation was calculated using Pearson's coefficients. A p-value of < 0.05 was considered to be statistically significant.

### Results

### Antibody responses to serotype 1 :

Mice were immunized i.n. with either 0.5 µg or 2.0 µg PNC-1 or PPS-1, with or without the mucosal adjuvant RV. Intranasal immunization with both 0.5 µg and 2.0 µg PNC-1 in saline elicited very low systemic responses, but the IgG levels were significantly higher than in unimmunized control mice (p<0.001). There was a highly significant increase in systemic IgG response when PNC-1 was mixed with RV (p<0.001, figure 1 and table 1 hereinafter) and immunization with 2.0 µg PNC-1 in RV elicited a significantly higher systemic IgG response than 0.5 µg PNC-1 (p=0.003). In preliminary studies, we observed that s.c. immunization with 0.5 µg PNC-1 elicited the highest antibody response and thus this dose was used. 0.5 µg PNC-1 in saline administrated s.c. elicited a higher IgG response than the same dose administrated i.n. with RV (p=0.038), but 2.0 µg PNC-1 in RV induced the highest antibody response of all immunized groups. Intranasal immunization with PPS-1 in RV or saline elicited very low IgG antibody responses, although the levels were significantly higher than in unimmunized control mice (p<0.001 and p=0.004, respectively) they were significantly lower than after i.n. immunization with PNC-1 (p<0.001). Only those mice immunized with PNC-1 s.c. (p=0.008) and PPS-1 in RV i.n (p=0.001) showed significant IgM responses compared to unvaccinated control mice (table 1). Significant systemic IgA responses (table 1) were only elicicted in mice immunized i.n. with 2.0 µg PNC-1 in RV (p<0.001), i.n. with PPS-1 in RV (p=0.030) and s.c with PNC-1 in saline (p=0.014). Moreover, a significantly higher systemic IgA response was observed for the RV group than the group immunized s.c. (p<0.001).

### Antibody responses to serotype 3 :

Mice were immunized with 2.0 µg of PNC-3, i.n. in saline or RV, or i.p. in FCA/FIA as PNC-3 is poorly immunogenic when given s.c. in saline (data not shown). Intraperitoneal immunization with PNC-3 emulsified with FCA/FIA elicited by far the highest type 3-specific IgG antibody titer in serum (figure 2). When PNC-3 was administrated i.n. with RV, a significant serum IgG antibody response was observed (p=0.003). Intranasal immunization with PNC-3 alone or with PPS-3 with or without RV did not induce significant IgG responses.

### Protection against pneumococcal infection caused by serotype 1 :

To evaluate the efficacy of the PNC-1 against bacteremia and pulmonary infection, mice were challenged intranasally with 4x10⁶ CFU serotype 1 pneumococci in 50 µl saline two weeks after booster immunization. Serotype 1 was very virulent and caused severe lung infection (mean = 7.70 log CFU/ml lung homogenate, figure 3a) and bacteremia (mean = 5.50 log CFU/ml blood, figure 3b) in unimmunized control mice 24 hours after i.n. challenge.

Immunization with PNC-1 conferred protection against lung infection caused by serotype 1 (figure 3a). Bacteria were cultured from lung homogenate of all unimmunized control mice 24 hours after challenge, whereas all mice immunized parenterally with PNC-1 were completely protected. In addition, 100% protection in lungs was observed in the group that received 2.0 µg PNC-1 with RV i.n. Mice that received 0.5 µg PNC-1 mixed with RV had significantly reduced pneumococci in lungs compared to unimmunized control mice (p<0.001) and 7/10 were fully protected. Both groups of mice immunized i.n. PNC-1 in saline had reduced pneumococcal density in lungs compared to unimmunized control mice (p<0.001) and of those receiving 2.0 µg PNC-1 3/10 were completely protected. In contrast, pneumococci were cultured from the lungs of all mice immunized i.n. with 0.5 µg PNC-1 in saline. All mice immunized i.n. with PPS-1 in saline were heavily infected in the lungs, but mice immunized i.n. with PPS-1 mixed with RV had reduced pneumococcal density (p<0.001) and 4/10 mice were protected.

Similarly, immunization with PNC-1 protected against pneumococcal bacteremia (figure 3b) and CFU in blood correlated with CFU in lung homogenate (r=0.852, p<0.001). Whereas all control mice had severe bacteremia, 100% protection was observed for the two groups that received PNC-1 mixed with RV i.n., the parenterally immunized group and the group immunized i.n with 2.0 µg PNC-1 in saline (figure 3b). Of the mice immunized i.n. with 0.5 µg PNC-1 in saline, 6/10 were protected against bacteremia. Intranasal administration of PPS-1 in RV gave 100% protection from bacteremia, but 5/10 mice immunized i.n. with PPS-1 in saline had detectable pneumococci in the blood.

The relationship between type 1-specific serum IgG antibodies and pneumococcal density in lungs and blood is shown in figure 4. Unimmunized mice had hardly detectable IgG antibodies and were heavily infected. Protection against lung infection was significantly corrrelated with type 1-specific IgG and IgA antibody levels in serum (r=-0.44, p<0.001 and r=-0.350, p=0.002 for IgG and IgA respectively) and in all mice with >1000 EU/ml IgG, pneumococci were not detectable in the lungs (figure 4a). However, ∼100 EU/ml IgG in serum was sufficient for protection against bacteremia (figure 4b).

### Protection against pneumococcal infection caused by serotype 3 :

It is already known that serotype 3 is virulent and causes severe lung infection in mice after i.n. challenge. Challenge with >10⁷ CFU of this serotype may also cause bacteremia, which kills the mice in 1-2 days. However, by reducing the challenge dose, survival may be prolonged. Thus, the mice were challenged i.n. with 4.5x10⁴ CFU of type 3 pneumococci in 50 µl saline and survival recorded over 7 days when the experiment was terminated (figure 5). Seven days after pneumococcal challenge only 30% of unvaccinated control mice had survived. There was no difference in survival at day 7 between the control mice and mice immunized i.n. with PNC-3 in saline (30%) and PPS-3 in saline (40%) or RV (20%). However, 8/10 mice immunized i.n. with PNC-3 in RV (p=0.006) and 9/10 mice immunized i.p. with PNC-3 in FCA/FIA (p=0.0003) survived and looked healthy at day 7. Nevertheless, when sacrificed at this time point, low levels of pneumococci (4x10²- 4x10⁴ CFU/ml lung homogenate) were detectable in the lungs of all mice.

Although there was a highly significant difference in serum IgG antibody levels between the i.n. and i.p. immunized mice (p<0.001), there was no difference in survival depending on the immunization route (p=0.290), indicating that the low titers of serum IgG antibodies elicited by i.n. immunization (figure 2 and table 1) were sufficient to protect the mice from severe pneumococcal lung infection by type 3.

### Conclusion

For PNC-1, i.n. immunization with RV was as efficient as immunization by the s.c. route, both in terms of immunogenicity and protection against pneumococcal pneumonia and bacteremia. Even though i.n. immunization with the corresponding polysaccharide PPS-1 in RV elicited a significant systemic IgG response, this only leads to partial protection against pulmonary infection. Thus, among the doses tested, PPS was less effective than PNC for mucosal immunization against pneumococcal infections.

Intranasal immunization with PNC-3 was compared with PNC-3 in FCA/FIA given i.p. Although the i.n. route induced a significantly lower systemic IgG response compared to the i.p. route, it reduced the severity of infection caused by type 3 pneumococci and prolonged survival to a similar degree.

Systemic IgG antibodies to PPS are known to correlate with protection against pneumococcal infections. In this study we demonstrated that i.n. immunization with PNC-1 and PNC-3 in RV protected mice against infection after i.n. challenge with the respective pneumococcal serotypes and that the protection was related to the levels of type specific serum IgG and IgA antibodies. These results indicate that mucosal vaccination with pneumococcal polysaccharide conjugate vaccines may be an alternative approach to current strategies for prevention against pneumococcal diseases.

**Table 1:**

| Type-specific antibodies in sera after immunization with pneumococcal polysaccharides (PPS) and pneumococcal conjugate vaccines (PNC) | | | | | | | | | | |
|---|---|---|---|---|---|---|---|---|---|---|
| | | | | | IgG | | IgM | | IgA | |
| | Vaccine | µg | Route | Adjuvant | GMT¹ | CI²⁾ | GMT¹ | CI²⁾ | GMT¹ | CI²⁾ |
| *Type 1* | | | | | | | | | | |
| A | PNC-1 | 0.5 | i.n. | RV | 771 | 384-1550 | 40 | 26-62 | 42 | 29-62 |
| B | PNC-1 | 2.0 | i.n. | RV | 5365 | 2720-10583 | 55 | 33-91 | 341 | 207-564 |
| C | PPS-1 | 2.0 | i.n. | RV | 38 | 11-125 | 71 | 47-106 | 52 | 37-72 |
| D | PNC-1 | 0.5 | i.n. | saline | 33 | 15-74 | 11 | 5-24 | 26 | 21-33 |
| E | PNC-1 | 2.0 | i.n. | saline | 220 | 132-371 | 23 | 13-43 | 49 | 28-85 |
| F | PPS-1 | 2.0 | i.n. | saline | 7 | 5-11 | 33 | 22-48 | 45 | 28-72 |
| G | PNC-1 | 0.5 | s.c. | saline | 2562 | 882-7441 | 99 | 31-312 | 62 | 40-95 |
| H | | | | control | 3 | 3-4 | 17 | 10-28 | 28 | 20-42 |

| *Type 3* | | | | | | | | | | |
|---|---|---|---|---|---|---|---|---|---|---|
| A | PNC-3 | 2.0 | i.n. | RV | 48 | 12-193 | 68 | 50-92 | | |
| B | PPS-3 | 2.0 | i.n. | RV | 5 | 4-6 | 49 | 34-70 | | |
| C | PNC-3 | 2.0 | i.n. | saline | 3 | 1-6 | 37 | 15-78 | | |
| D | PPS-3 | 2.0 | i.n. | saline | 4 | 3-8 | 44 | 16-121 | | |
| E | PNC-3 | 2.0 | i.p. | FCA/FIA | 1650 | 1131-2407 | 147 | 67-327 | | |
| F | | | | control | 2 | 2-3 | 38 | 15-97 | | |

| | | | | | | | | | | |
|---|---|---|---|---|---|---|---|---|---|---|
| 1) GMT; genometric mean titers (EU/ml) | | | | | | | | | | |
| 2) CI; 95% confidence intervals | | | | | | | | | | |

### Abbreviations

CFU : colony forming units ; CT : cholera toxin ; CTB : cholera toxin B subunit; CWPS : cell wall polysaccharide ; ELISA : enzyme-linked immunosorbent assay ; EU : Elisa Units ; FA : Freund's adjuvant; FCA : Freund's complete adjuvant; FIA : Freund's incomplete adjuvant ; i.n. : intranasal ; i.p. : intraperitoneal ; LT : *Escherichia coli* heat-labile enterotoxin ; MALT : mucosal associated lymphoid tissue ; PBS : phosphate-buffered saline ; PNC : pneumococcal polysaccharide conjugate vaccine ; PPS : pneumococcal polysaccharides ; RV: RhinoVax ; s.c.; subcutaneous ; TI-2 : thymus-independent antigen type 2.

## Claims

1. The use of a *Streptococcus pneumoniae* capsular polysaccharide in the preparation of a medicament to be mucosally administered for preventing against diseases induced by *S. pneumoniae* infections.

2. The use according to claim 1, of a *S. pneumoniae* capsular polysaccharide in the preparation of a medicament to be intranasally administered.

3. The use according to claim 2, of a *S. pneumoniae* capsular polysaccharide in the preparation of a medicament to be intranasally administered so that the medicament is essentially delivered to the respiratory tract.

4. The use according to claim 1, 2 or 3, of a *S. pneumoniae* capsular polysaccharide which is conjugated to a carrier protein.

5. The use according to claim 4, of a *S. pneumoniae* capsular polysaccharide which is conjugated to a carrier protein which is selected from the group consisting of the tetanus or diphtheriae toxoid.

6. The use according to any one of claims 1 to 5, of a *S. pneumoniae* capsular polysaccharide in combination with a mucosal adjuvant.

7. The use according to claim 6, of a *S. pneumoniae* capsular polysaccharide in combination with a mucosal adjuvant selected from the group consisting of cholera toxin, a subunit thereof, a mutant thereof, *E. coli* heat-labile toxin, a subunit thereof, or a mutant thereof.

8. The use according to claim 6, of a *S. pneumoniae* capsular polysaccharide in combination with a mucosal adjuvant selected from the group consisting of the compounds described in claim 1 of W094/17827 as published on Aug. 18, 1994.

9. The use according to claim 8, of a *S. pneumoniae* capsular polysaccharide in combination with a mucosal adjuvant which is RhinoVax™.

10. The use according to any one of claims 1 to 9, of a capsular polysaccharide of *S. pneumoniae* serotype 1, 3, 4, 5, 6B, 7F, 9V, 14, 18C, 19F or 23F.
